(19) **Europäisches Patentamt / European Patent Office / Office européen des brevets**

(11) **EP 4 624 924 A1**

(12) # EUROPEAN PATENT APPLICATION

(43) Date of publication:
01.10.2025 Bulletin 2025/40

(21) Application number: 24165999.4

(22) Date of filing: 25.03.2024

(51) International Patent Classification (IPC):
**G01N 33/38** (2006.01)

(52) Cooperative Patent Classification (CPC):
(C-Sets available)
**G01N 33/38; C04B 28/065; C04B 38/02;**
C04B 2111/28; C04B 2111/52 (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Sika Technology AG**
**6340 Baar (CH)**

(72) Inventors:
• **KOCH, Lukas**
**83454 Anger (DE)**
• **MOHRI, Nils**
**83308 Trostberg (DE)**

(74) Representative: **Sika Patent Attorneys**
**C/o Sika Technology AG**
**Corp. IP Dept.**
**Tüffenwies 16**
**8048 Zürich (CH)**

(54) **METHOD AND SYSTEM FOR THE FORMULATION OR PRODUCTION OF A MINERAL FOAM**

(57) The present invention relates to computer implemented methods and systems to determine, based on given constituent parameters and environmental parameters, at least one formulation parameter and/or at least one process parameter of a mineral foam, suitable to achieve a given performance parameter.

**Figure 1**

**(Cont. next page)**

(52) Cooperative Patent Classification (CPC): (Cont.)

C-Sets
**C04B 28/065, C04B 7/02, C04B 7/32,**
**C04B 22/068, C04B 40/0042;**
**C04B 28/065, C04B 7/02, C04B 7/32, C04B 38/02,**
**C04B 40/0042;**
**C04B 38/02, C04B 28/02, C04B 28/06,**
**C04B 28/065**

## Description

### Technological field

The present invention relates to computer-implemented methods to determine formulation parameters and/or process parameters of mineral foams.

### Background of the invention

It is well known that mineral foams can be used as insulation material, e.g., as a thermal insulator, acoustic insulator or acoustic absorber as well as construction material with a low density. In contrast to foams based on organic polymers, mineral foams are eco-friendly, robust, and non-flammable.

Many methods are known from the prior art to produce inorganic foams. For example, WO 01/70647 (Windsor Technologies Ltd) discloses a method where a dry mineral binder is added to a pre-generated aqueous foam. For example, WO 2019/038105 (BASF SE) discloses a method where a cement slurry and an aqueous foam are separately prepared and then mixed. For example, WO 2018/162381 (Construction Research & Technology GmbH, DeCavis AG) discloses a method where a mineral foam is obtained by chemical foaming of a mineral binder slurry. All of these methods rely on the introduction and/or generation of gas in a wet system with subsequent drying and/or curing.

Mineral foams are based on mineral binders and typically contain a gas-filled cell structure. One particularly important performance parameter is dry mineral foam density. Dry mineral foam density largely depends on the amount of the gas-filled cells. Other parameters, such as mechanical strength or insulation properties in turn depend on the dry mineral foam density.

When producing a mineral foam according to any of the methods of the prior art it is relevant to set and to control the overall formulation of the mineral foam. It is likewise necessary to set and to control the process by which the mineral foam is formed. Especially, this is necessary to determine and to control the amount of water as well as the amount and dispersion of gas in the mineral foam. Thereby, in particular the dry mineral foam density can be controlled.

Water as well as gas are added, consumed, and/or removed in every step of the production of a mineral foam. For example, when preparing a slurry of a mineral binder, water is added, and gas is entrained depending on the mixing process. Addition of a foaming agent may add more water (depending on the formulation of the foaming agent) and leads to the generation of gas in the mix. Additionally, the mineral foam temperature and the environmental pressure each have an influence on the gas expansion and/or gas volume. Upon curing, the mineral binder used to prepare the foam typically consumes water and hardens thereby. Additionally water may evaporate and/or may be actively removed.

It is therefore particularly difficult to determine and to control the amounts of water and gas in a mineral foam. This frequently leads to varying performance of mineral foams and to increased need for lab testing.

It would therefore be desirable to have available improved methods to determine formulation parameters and/or process parameters of mineral foams.

### Summary of the invention

It as an objective of the present invention to provide computer implemented methods for the determination of formulation parameters and/or process parameters of mineral foams. Thereby, mineral foams could be produced to fulfill desired performance parameters, such as dry mineral foam density and/or dry mineral foam volume.

It is another objective of the present invention to provide methods for the production of mineral foams, whereby formulation parameters and/or process parameters of the mineral foam are determined using a computer implemented method.

These and other objectives are solved by the subject matter of the independent claims.

Preferred embodiments are the subject matter of dependent claims.

It was in particular possible to provide a computer implemented method that, based on input parameters related to desired performance of the mineral foam, input parameters related to the chemical and/or physical properties of constituents of the mineral foam, and input parameters related to environmental conditions, can determine formulation parameters and/or process parameters of the mineral foam. Formulation parameters and/or process parameters thus determined are suitable to produce a mineral foam with the desired performance.

It is an advantage of the methods of the present invention that the formulation parameters and/ process parameters do not have to be established by a large number of lab trials, thereby saving time. It is another advantage that the formulation parameters and/ process parameters can be easily and quickly determined and thereby for example adapted to continuously changing inputs, such as changes in constituents of the mineral foam, changes in environmental conditions, or changing performance requirements. Thereby, production of mineral foams having desired performance is made particularly simple.

## Detailed ways

In a first aspect the present invention relates to a computer implemented method to determine at least one formulation parameter and/or at least one process parameter of a mineral foam, said method comprising the steps of

(i) capturing and/or determining

-    at least one performance parameter of the mineral foam,
-    at least one constituent parameter characterizing a chemical and/or physical property of at least one constituent of the mineral foam,
-    at least one environmental parameter,

(ii) providing the parameters captured and/or determined in step (i) to a predictive model,
(iii) determining, using the predictive model, at least one formulation parameter and/or at least one process parameter of the mineral foam, suitable to achieve the at least one performance parameter with the given constituent parameters and environmental parameters, and
(iv) outputting the at least one formulation parameter and/or at least one process parameter determined in step (iii) via a user interface, a machine interface, and/or on a data storage device.

A mineral foam within the present context is a two-phase or three-phase system. Thereby, one phase is gaseous, one phase is solid, and optionally a liquid phase is additionally present. The gaseous phase is present as fine gas bubbles separated by cell walls thereby forming a cell structure. The cell walls are the solid phase and are obtained by the curing reaction of mineral binders with water. The content of the gaseous phase in the mineral foam can vary in a range from 20 to 99% or 20 to 98% by volume, preferably from 50 to 98% by volume. Water may be additionally present as a liquid phase.

Mineral foams can be present in a wet form. Wet mineral foams contain a significant amount of water, and typically the mineral binder is not cured or not fully cured. For example, a wet mineral foam can contain water in a weight ratio of water to solids between 0.4 - 2.0, preferably 1.0 - 1.6.

Mineral foams can be present in a dry form. Dry mineral foams contain no water or small amounts of water, and the mineral binder is at least partly, especially fully cured. Water present in particular is water adsorbed to cured mineral surface of cell walls. It is preferred that a dry mineral foam contains not more than 5 w%, especially not more than 1 w%, relative to the total weight of the dry mineral foam, of water.

Mineral binders are inorganic materials that can react with water to form water insoluble hydrate phases. According to embodiments, mineral binders are selected from cements, pozzolanes, latent hydraulic materials, lime, calcium sulfate, or mixtures thereof.

Cements preferably are selected from Portland cements according to according to standard EN 197-1:2011, alumina cement, especially alumina cement according to standard EN 14647:2005, calcium sulphoaluminate cement, or mixtures thereof.

Pozzolanes and latent hydraulic binders preferably are selected from slag, especially blast furnace slag or basic oxygen slag, clay, calcined clay, especially metakaolin, kiln dust, microsilica, fly ash, pyrogenic silica, precipitated silica, silica fume, zeolite, rice husk ash, burnt oil shale, natural pozzolane such as pumice and trass, or mixtures thereof.

Lime is preferably selected from natural hydraulic lime, formulated lime, hydraulic lime, and air lime according to standard EN 459-1:2015.

Calcium sulfate preferably is selected from anhydrite, calcium sulfate hemihydrate, calcium sulfate dihydrate, gypsum, or mixtures thereof.

According to embodiments, the at least one formulation parameter and/or the at least one process parameter are selected from parameters having an influence on the total amount of gas present in the mineral foam.

This allows in particular to control the dry mineral foam density and/or dry mineral foam volume or to respond to a given dry mineral foam density and/or dry mineral foam volume determined and/or captured as performance parameter in step (i).

The expression "constituents of the mineral foam" refers to raw materials or intermediates used in the preparation of a dry mineral foam of the present invention. Constituents comprise at least one mineral binder, a gas, water, and optionally further additives. Constituents can be in the form of a dry premix, an aqueous slurry, an aqueous foam, or a wet mineral foam.

The expression "gas" refers to all materials that are in a gaseous state at 25 °C and 1023 mbar. Preferred gases are hydrogen, nitrogen, oxygen, carbon dioxide, noble gases in particular argon, krypton, and xenon, or gas mixtures such as air. Air is particularly preferred within the present context.

Further additives that may optionally be present in particular are aggregates, fillers, and admixtures. Especially, aggregates, fillers, and admixtures which are commonly used in the formulation of mortars and concrete can be used.

Fillers are inert mineral particles in finely divided form. Fillers may also be stabilizing particles. Admixtures can for example be plasticizers or superplasticizers such as polycarboxylate ethers, accelerators, retarders, shrinkage reducing agents, fibers, foaming agents, surfactants, foaming powder, pigments, and/or biocides.

Stabilizing particles are particles that can stabilize a mineral foam, in particular by pickering. Suitable median particle sizes D50 of stabilizing particles range from 30 nm to 300 $\mu$m, as measured by dynamic light scattering according to standard ISO 22412:2008.

An aqueous foam comprises water and surfactants and/or stabilizing particles suitable to stabilize the foam. An aqueous foam within the present context does not contain a mineral binder.

Suitable surfactants are non-ionic, anionic, cationic, zwitterionic, and amphiphilic compounds as well as proteins or mixtures thereof. Particularly suitable are C8-C18-alkyl sulfates, C8-C18-alkyl ether sulfates, C8-C18-alkyl sulfonates, C8-C18-alkyl benzene sulfonates, C8-C18-$\alpha$-olefin sulfonates, C8-C18-sulfosuccinates, $\alpha$-sulfo-C8-C18-fatty acid salts, C8-C18-fatty acid salts, C8-C18-fatty alcohol ethoxylates, block copolymers of ethylene oxide and propylene oxide, C8-C18-alkyl polyglycosides, alkyltriammonium salts, alkylbenzyldimethylammonium salts, alkylpyridinium salts, cocamidopropyl betaine, lauramidopropyl betaine, keratin, hydrolyzed keratin, collagen, hydrolyzed collagen, or soy-based proteins.

It is preferred, that all dry constituents of a mineral foam of the present invention are present in the form of a dry premix.

In an exemplary embodiment, the constituents of the wet mineral foam are:

- a dry premix of mineral binder, aggregate, stabilizing particles, foaming powder, surfactant, retarder, accelerator, superplasticizer, and shrinkage reducing agent, whereby the foaming powder is suitable to catalyze the decomposition of a foaming agent; and
- an aqueous solution of a foaming agent, for example hydrogen peroxide.

In another exemplary embodiment, the constituents of the wet mineral foam are:

- an aqueous slurry of mineral binder, aggregate, filler, and superplasticizer; and
- an aqueous foam comprising a surfactant and/or foaming agent.

Throughout the present context the expressions "slurry" and "suspension" are used interchangeably.

According to embodiments, the constituents of the mineral foam are selected from one or more of a dry premix of constituents, water, a mineral binder slurry, a foaming agent, an aqueous suspension of dry premix of constituents, an aqueous foam, and/or a wet mineral foam.

Preferably, the dry premix of constituents comprises the mineral binder and, if present, aggregates, fillers, and admixtures. Admixtures preferably are superplasticizers, accelerators, retarders, shrinkage reducing agents, and foaming powder.

A foaming powder is a material suitable to catalyze the decomposition, and thereby gas formation, of a foaming agent. Suitable foaming powders preferably comprise salts of Mn(II), Mn(IV), Mn(VII) or Fe(III). Alternatively, the enzyme catalase may be used as foaming powder. Non-limiting examples of foaming powders are $MnO_2$ and $KMnO_4$. Such foaming powders are preferably used in combination with peroxide foaming agents.

A foaming agent is a material that evaporates, decomposes, or reacts with water and/or an acid, so as to liberate the gas. Non-limiting examples of foaming agents are peroxides, such as hydrogen peroxide, dibenzylperoxide, peroxobenzoic acid, peroxoacetic acid, alkali metal peroxides, perchloric acid, peroxomonosulfuric acid, dicumyl peroxide or cumyl hydroperoxide; isocyanates; carbonates and bicarbonates, such as $CaCOs$, $Na_2CO_3$, and $NaHCOs$, which are preferably used in combination with an acid, e.g. a mineral acid; metal powders, such as aluminum powder; azides, such as methylazide; hydrazides, such as p-toluenesulfonylhydrazide; hydrazine. The foaming agent preferably is present in dissolved or dispersed form, especially in the form of an aqueous solution.

A mineral binder slurry is an aqueous slurry of at least one mineral binder.

An aqueous suspension of dry premix of constituents can, for example, be an aqueous slurry of mineral binder, or an aqueous suspension of a premix of mineral binder, aggregate, filler, and optionally foaming powder. Further additives as defined above may also be present in an aqueous suspension of dry premix of constituents.

An aqueous foam is as described above.

A wet mineral foam is as described above.

A wet mineral foam can, for example, be produced by mixing an aqueous foam with an aqueous suspension of dry premix of constituents. A wet mineral foam can, for example, be produced by chemically and/or physically foaming an aqueous suspension of dry premix of constituents.

A constituent parameter is a parameter describing a chemical and/or physical composition or property of the respective constituent.

According to embodiments, the at least one constituent parameter is selected from the density of the foaming agent, the decomposition rate of the foaming agent, the concentration of water in the foaming agent, the amount of water needed to cure the mineral binder, the water to solids weight ratio in the aqueous suspension of dry premix of constituents, the density of the aqueous suspension of dry premix of constituents, the volume of the aqueous suspension of dry premix of constituents, the water to solids weight ratio in the aqueous foam, the density of the aqueous foam, the water to solids weight ratio in the wet mineral foam.

In particular, the at least one formulation parameter is selected from the respective weight ratios of the dry premix of constituents, water, foaming agent, the weight ratio of any of additive present, especially any of retarder, accelerator, superplasticizer, and shrinkage reducing agent present, the volume of the foaming agent, and/or the volume of the aqueous foam.

Weight ratios in particular are weight-% of the respective constituent relative to the total weight of the wet mineral foam.

Where the foaming agent is present in dispersion or solution, the volume of the foaming agent refers to the total volume of such dispersion or solution.

The amount of water needed to cure the mineral binder is the amount of water that is chemically bound or consumed during the hardening of the mineral binder. For example, the amount of water needed to cure the mineral binder is given in weight % relative to the mineral binder or relative to the dry premix of constituents comprising the mineral binder.

In particular, the at least one process parameter is selected from the mixing speed and/or mixing time of constituents, the area of a phase boundary between gas and mineral binder slurry, or between gas and aqueous suspension of dry premix of constituents, or between gas and aqueous foam, the relative pump speed of the foaming agent, the relative pump speed of the aqueous foam, the wet mineral foam volume.

The expression "pump speed" relates to the speed of dosing equipment used for providing the respective constituent to production equipment. Thereby, the expression "pump speed" is correlated to the speed of dosing the respective material. In particular, the pump speed of constituents is provided relative to the pump speed of other constituents. Therefore, 100% relative pump speed means that the dosing pumps operate to dose equal volumes of constituents.

The gaseous phase of a mineral foam of the present invention can be introduced by mechanical foaming in the presence of the respective gas. Mechanical foaming may be performed in a batch process or in a continuous process. Mixing can be static or dynamic. The gaseous phase can also be introduced into a mineral foam of the present invention by physical or chemical foaming, wherein the physical or chemical foaming process is suitable to liberate a gas. Preferably, foaming agents are used, which evaporate, decompose or react with water and/or an acid, so as to liberate the gas.

According to embodiments, the at least one performance parameter of the mineral foam is the dry mineral foam density and/or dry mineral foam volume.

For example, the dry mineral foam density can be measured according to standard EN 1602:2013-05.

According to embodiments, the at least one environmental parameter is selected from the weight loss upon drying, the wet mineral foam temperature, and/or the atmospheric pressure.

Capturing and/or determining of the parameters in step (i) is for example performed by manually inputting the respective parameters, reading in the respective parameters via a machine interface and/or by reading the respective parameters with sensors.

A sensor is to be understood in a broad sense and stands for a device capable of determining a certain parameter in step (i). A sensor in particular is chosen from a sensor capable of capturing or determining a chemical and/or physical property of at least one individual constituent of the mineral foam, and/or from a sensor capable of capturing or determining environmental parameters.

For example, a sensor capable of measuring environmental conditions in particular is chosen from a sensor capable of determining a temperature, humidity, solar irradiance, air pressure, wind speed, wind direction, atmospheric composition, and/or altitude.

Preferably, in a method of the present invention, the at least one environmental parameter is measured by a sensor.

Furthermore, capturing and/or determining of the parameters in step (i) may be effected by reading in the respective parameters via a machine interface. The parameters captured and/or determined in this manner may be obtained from sensors and/or in the form of processed data, e.g. data provided online in a computer network, for example data provided on an internet website. Especially, in step (i), at least one parameter, especially the at least one environmental parameter, is measured with a sensor and/or calculated from sensor data.

Especially for manually inputting data, a user interface that queries at least one parameter, especially all of the parameters, may be provided. In this case, in step (i), the parameters can be captured via a user interface, especially via a graphical user interface. Thereby, in particular, the user interface is configured such that for at least one parameter, especially for all parameters, a predefined list of selectable parameters is presented to a user.

Especially, the parameters of the present invention are represented by a numerical value. Nevertheless, a parameter may be represented in the form of a string and/or a symbol and later assigned a numerical value. This may in particular facilitate the manual capturing of the respective parameters for users.

The predictive model used in step (iii) may be a set of functional relationships based on multivariable functions. Thereby, the at least one formulation parameter and/or at least one process parameter are functionally depending on the at least one performance parameter, at least one constituent parameter, and/or at least one environmental parameter.

Preferably, the functional relationship is based on linear combinations or series expansion equations of on the at least one performance parameter, at least one constituent parameter, and/or at least one environmental parameter. Particularly suitable series expansion equations are Taylor series. A linear combination is meant to be an expression constructed from a set of terms by multiplying each term by a constant and adding the results.

For example, a suitable functional relationship may be defined as follows:

$$FP_i = c_0 + a_i \cdot PP + b_i \cdot CP + c_i \cdot EP \text{ and/or } PrP_i = c_0 + a_i \cdot PP + b_i \cdot CP + c_i \cdot EP$$

with $FP_i$ = i-th formulation parameter, PrPi = i-th process parameter, whereby i = 1, 2, 3, ...; $c_0$ = constant value; $a_i$, $b_i$, $c_i$ = coefficients of linear combination; PP = performance parameter represented by a numerical value; CP = constituent parameter represented by a numerical value; EP = environmental parameter represented by a numerical value.

However, other functional relationships may be used as well.

In particular, before step (i) the functional relationships are obtained, especially with a regression analysis. The functional relationships, especially constants of the functional relationships, are in particular obtained by performing a regression analysis on a set of data points comprising formulation parameters and/or process parameters, the at least one constituent parameter, and the at least one environmental parameter, as independent variables and measured performance parameters as dependent variables.

However, other approaches for determining the functional relationships can be used as well, e.g. machine learning approaches.

The predictive model can also be a deep learning model and/or include at least one neural network. The predictive model can form part of an artificial intelligence system.

According to embodiments, the predictive model is selected from classification algorithms or regression algorithms, preferably from linear classifiers, support vector machines, maximum likelihood classifiers, decision trees, K-nearest neighbors, random forests, linear regression models, logistical regression models, and/or polynomial regression models.

According to embodiments, the predictive model is selected from clustering algorithms, preferably hierarchical clustering, K-means clustering, Bayes nets, Gaussian mixture models, and/or dimensionality reduction models.

The predictive model may output the result of analysis via a user interface, a machine interface, and/or on a data storage medium. Thereby, the output can take the form of a text-based answer to a question, a symbol, or a number, preferably attached with a respective unit.

It is possible that a method of the present invention additionally comprises a step of (v) running a simulation to predict at least one performance parameter of the mineral foam based on the at least one constituent parameter and the at least one environmental parameter as well as the at least one formulation parameter and/or the at least one process parameter.

In particular, for such simulation, the predictive model uses the at least one formulation parameter and/or the at least one process parameter determined in steps (i) - (iv) of the inventive method. Thereby, it is possible to confirm that the performance parameter captured and/or determined in step (i) is actually met by the determined formulation parameters and/or process parameters.

A simulation is in particular suitable where a plurality of formulation parameters and/or process parameters is outputted. The method may then additionally comprise a step of selecting at least one specific formulation parameter and/or at least one specific process parameter. Such selection can be based on a simulation result. Formulation and/or process parameters may be selected that best approximate the desired performance parameters.

The method of the present invention can be implemented in a flexible manner with known software architectures, e.g. as native application, a progressive web application (PWA), or a hybrid application (combination of native and PWA). Thereby, helpful internet links to tutorials or support sites as well as sharing functions (e.g. via e-mail, Bluetooth, Airdrop, or others communication means) can be included in such applications as well. Also, the inventive method can be implemented in one single application, or it may be divided into two or more separate applications with appropriate software interfaces for data exchange between the applications. Applications can be implemented for any kind of operating systems such as e.g. iOS, Android, Microsoft Windows and/or Linux.

In another aspect, the present invention relates to a non-transitory computer-readable medium storing instructions that, when executed by one or more processors, cause the one or more processors to run a method as described above.

All features and embodiments as described above also apply to this aspect.

Examples of a computer-readable storage medium may include any tangible media capable of storing electronic data, including volatile memory or non-volatile memory, removable or non-removable memory, erasable or non-erasable memory, writeable or rewriteable memory, and so forth. Embodiments may also be at least partly implemented as instructions contained in or on a non-transitory computer-readable medium, which may be read and executed by one or more processors to enable performance of the operations described herein.

According to embodiments, the non-transitory computer-readable medium may be selected from hard disk drives, solid state drives, CD-ROM, DVD, Blu-ray discs, USB

Flash Drives, memory cards, magnetic tapes, floppy disks, EPROM, optical drives, magnetic diskettes, compact flash cards, secure digital cards, external hard drives, network attached storage, cloud storage, RAID, 3D XPoint memory.

The invention further includes an apparatus comprising a non-transitory computer-readable storage medium storing logic for a predictive model suitable to determine the at least one formulation parameter and/or the at least one process parameter, optionally a hardware interface configured to receive training data and a hardware processor circuit configured to train the predictive model based on the training data.

According to embodiments, the non-transitory computer-readable medium further stores instructions for the production of the mineral foam, whereby the at least one formulation parameter and/or at least one process parameter determined is used for said production.

In particular, instructions for the production of mineral foam can be instructions to control dosing equipment of a production facility, such as a mixer, and/or instructions to control heating or cooling devices attached to a production facility.

In another aspect, the present invention relates to a system comprising

(a) means for capturing and/or determining

- at least one performance parameter of a mineral foam,
- at least one constituent parameter characterizing a chemical and/or physical property of at least one constituent of the mineral foam,
- at least one environmental parameter,

(b) means for providing the parameters captured and/or determined to a predictive model,

(c) a predictive model, configured and/or trained to determine at least one formulation parameter and/or at least one process parameter of the mineral foam, suitable to achieve the at least one performance parameter with the given constituent parameters and environmental parameters, and

(d) means for making available the at least one formulation parameter and/or at least one process parameter in particular a user interface, a machine interface, and/or a data storage device.

All features and embodiments as described above also apply to this aspect.

The inventive system can for example be any kind of computer device and/or control unit.

According to a special embodiment, the system is a mobile computer device. A mobile computer device in particular is meant to be a handheld computer, i.e. a computer small enough to hold and operate in the hand of a human. Especially selected from a mobile phone, a mobile computer or a portable computer. Thereby, for example, the system can be used directly on a construction site.

The system can be implemented in the form of a standalone application running on the mobile device and/or the control unit of a machine without need for any further resources such as for example server systems. This is in particular helpful in areas with limited access to communication networks, e.g. far away from urban centers or underground. However, the system can be implemented as well in a distributed computing environment, e.g. comprising the mobile device and/or the control unit as a client in combination with a dedicated server as a storage unit and/or with a specialized processing unit.

In another preferred embodiment, the system is implemented within a control unit of a machine that is configured for producing mineral foams, such as e.g. a mixing device.

Especially the system is a control unit of a mineral foam production facility. Preferably, the system controls mixing machinery to produce the mineral foam. This may be possible by transmitting instructions configured to cause the mixing machinery to acquire and mix constituents specified in the output as formulation parameters and/or according to a process specified in the output as process parameters.

According to embodiments, the system as described above additionally comprises machinery configured for the production of a mineral foam.

Suitable machinery can, for example, be a mineral foam production facility such as a mixing device, dosing pumps, weight scales.

In another aspect, the present invention relates to a method for the production of a mineral foam, said method comprising

(i) a step of determining at least one formulation parameter and/or at least one process parameter of a mineral foam, whereby

- at least one performance parameter of the mineral foam, at least one constituent parameter characterizing a chemical and/or physical property of at least one constituent of the mineral foam, and at least one environmental parameter are captured and/or determined,
- the parameters captured and/or determined are provided to a predictive model,
- using the predictive model, at least one formulation parameter and/or at least one process parameter of the mineral foam, suitable to achieve the at least one performance parameter with the given constituent parameters and environmental parameters, is determined, and
- the at least one formulation parameter and/or at least one process parameter is outputted via a user interface, a machine interface, and/or on a data storage device;

(ii) a step of producing a mineral foam, whereby the at least one formulation parameter and/or at least one process parameter determined in step (i) is used for said production.

All features and embodiments as described above also apply to this aspect.

The at least one formulation parameter and/or the at least one process parameter may be displayed and, if judged acceptable, the mineral foam may be produced by a suitable mixing machinery.

**Figures**

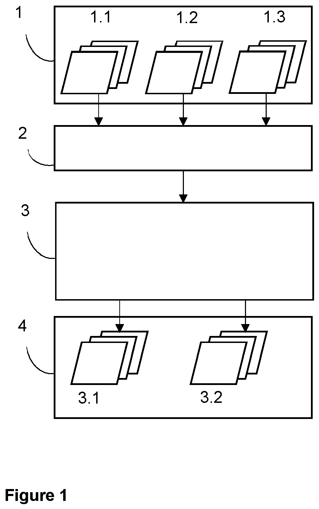

Figure 1 Figure 1 is a representation of a method of the present invention.

**Exemplary embodiments**

Figure 1 shows a method of the present invention. In a first step (1), at least one performance parameter of a mineral foam (1.1), at least one constituent parameter of a constituent of the mineral foam (1.2), and at least one environmental parameter (1.3) is captured and/or determined. In the next step (2), the parameters captured and/or determined are provided to a predictive model. In the next step (3), the predictive model, determines at least one formulation parameter (3.1) and/or at least one process parameter (3.2) of the mineral foam, suitable to achieve the performance parameters (1.1) with the given constituent parameters (1.2) and environmental parameters (1.3). The at least one formulation parameter (3.1) and/or at least one process parameter (3.2) of the mineral foam are outputted in the next step (4).

For example, to determine at least one formulation parameter and at least one process parameter of a mineral foam formed from an aqueous suspension of dry premix of constituents and a foaming agent, in step (1),

- a dry mineral foam density at 30°C and 20% r.h. of 70 kg/m$^3$ was captured as performance parameter (1.1) by manual input,
- a pre-mix dry mortar composition comprising mineral binder, aggregate, and a foaming powder (consisting of manganese(IV) oxide, calcium carbonate, and enanthic acid); a concentration of hydrogen peroxide aqueous solution of 30 w%; a water to solids weight ratio in the wet mineral foam of 1.125; an amount of water needed to cure the dry mortar of 23.27 w% relative to the dry mortar; a volume of the aqueous suspension of dry mortar and foaming powder of 45 L; and a density of the aqueous suspension of dry mortar and foaming powder of 1000 kg/m$^3$, were captured as constituent parameters (1.2) by manual input, and
- a wet mineral foam temperature of 35 °C and an atmospheric pressure of 1000 hPa were determined as environmental parameters (1.3) by measurement with a temperature and a pressure sensor respectively.

These captured and/or determined parameters were provided to a predictive model consisting of a set of series expansion equations in step (2). In a step (3) the predictive model determined

- a weight ratio of 47.6 w% of water, 46.1 w% of the pre-mix of dry mortar composition, and 6.3 w% of the 30% aqueous solution of hydrogen peroxide as formulation parameters (3.1), and
- a pump speed for the foaming agent of 6.05 % relative to the pump speed of the aqueous suspension of dry premix of constituents as process parameter (3.2).

In addition, the predictive model simulated a weight loss upon drying of the wet mineral foam of 41.99 w% and a dry mineral foam volume of 0.3898 m$^3$. The determined formulation parameters (3.1), performance parameter (3.2) and additional parameters determined were outputted via a user interface.

**Claims**

1. A computer implemented method to determine at least one formulation parameter and/or at least one process parameter of a mineral foam, said method comprising the steps of

   (i) capturing and/or determining

      - at least one performance parameter of the mineral foam,
      - at least one constituent parameter characterizing a chemical and/or physical property of at least one constituent of the mineral foam,
      - at least one environmental parameter,

   (ii) providing the parameters captured and/or determined in step (i) to a predictive model,
   (iii) determining, using the predictive model, at least one formulation parameter and/or at least one process parameter of the mineral foam, suitable to achieve the at least one performance parameter with the given constituent parameters and environmental parameters, and
   (iv) outputting the at least one formulation parameter and/or at least one process parameter determined in step (iii) via a user interface, a machine interface, and/or on a data storage device.

2. The method as claimed in any of the previous claims, **characterized in that** the at least one formulation parameter and/or the at least one process parameter are selected from parameters having an influence on the total amount of gas present in the mineral foam.

3. The method as claimed in any of the previous claims, **characterized in that** constituents of the mineral foam are selected from one or more of a dry premix of constituents, water, a mineral binder slurry, a foaming agent, an aqueous suspension of dry premix of constituents, an aqueous foam, and/or a wet mineral foam.

4. The method as claimed in any of the previous claims, **characterized in that** the at least one formulation parameter is selected from respective weight ratios of the dry premix of constituents, water, foaming agent, the weight ratio of any of additive present, especially any of retarder, accelerator, superplasticizer, and shrinkage reducing agent present, the volume of the foaming agent, and/or the volume of the aqueous foam.

5. The method as claimed in any of the previous claims, **characterized in that** the at least one process parameter is selected from the mixing speed and/or mixing time of constituents, the area of a phase boundary between gas and mineral binder slurry, or between gas and aqueous suspension of dry premix of constituents, or between gas and aqueous foam, the relative pump speed of the foaming agent, the relative pump speed of the aqueous foam, and/or the wet mineral foam volume.

6. The method as claimed in any of the previous claims, **characterized in that** the at least one performance parameter of the mineral foam is the dry mineral foam density and/or the dry mineral foam volume.

7. The method as claimed in any of the previous claims, **characterized in that** the at least one constituent parameter is selected from the density of the foaming agent, the decomposition rate of the foaming agent, the concentration of water in the foaming agent, the amount of water needed to cure the mineral binder, the water to solids weight ratio in the aqueous suspension of dry premix of constituents, the density of the aqueous suspension of dry premix of constituents, the volume of the aqueous suspension of dry premix of constituents, the water to solids weight ratio in the aqueous foam, the density of the aqueous foam, the water to solids weight ratio in the wet mineral foam.

8. The method as claimed in any of the previous claims, **characterized in that** the at least one environmental parameter is selected from the weight loss upon drying, the wet mineral foam temperature, and/or the atmospheric pressure.

9. The method as claimed in any of the previous claims, **characterized in that** the at least one environmental parameter is measured by a sensor.

**10.** The method as claimed in any of the previous claims, **characterized in that** it additionally comprises a step of (v) running a simulation to predict at least one performance parameter of the mineral foam based on the at least one constituent parameter and the at least one environmental parameter as well as the at least one formulation parameter and/or the at least one process parameter.

**11.** A non-transitory computer-readable medium storing instructions that, when executed by one or more processors, cause the one or more processors to run a method as claimed in any of claims 1-10.

**12.** The medium as claimed in claim 11, further storing instructions for the production of the mineral foam, whereby the at least one formulation parameter and/or at least one process parameter determined is used for said production.

**13.** A system comprising

(a) means for capturing and/or determining

- at least one performance parameter of a mineral foam,
- at least one constituent parameter characterizing a chemical and/or physical property of at least one constituent of the mineral foam,
- at least one environmental parameter,

(b) means for providing the parameters captured and/or determined to a predictive model,
(c) a predictive model, configured and/or trained to determine at least one formulation parameter and/or at least one process parameter of the mineral foam, suitable to achieve the at least one performance parameter with the given constituent parameters and environmental parameters, and
(d) means for making available the at least one formulation parameter and/or at least one process parameter in particular a user interface, a machine interface, and/or a data storage device.

**14.** The system according to claim 13, additionally comprising machinery configured for the production of a mineral foam.

**15.** A method for the production of a mineral foam, said method comprising

(i) a step of determining at least one formulation parameter and/or at least one process parameter of a mineral foam, whereby

- at least one performance parameter of the mineral foam, at least one constituent parameter characterizing a chemical and/or physical property of at least one constituent of the mineral foam, and at least one environmental parameter are captured and/or determined,
- the parameters captured and/or determined are provided to a predictive model,
- using the predictive model, at least one formulation parameter and/or at least one process parameter of the mineral foam, suitable to achieve the at least one performance parameter with the given constituent parameters and environmental parameters, is determined, and
- the at least one formulation parameter and/or at least one process parameter is outputted via a user interface, a machine interface, and/or on a data storage device;

(ii) a step of producing a mineral foam, whereby the at least one formulation parameter and/or at least one process parameter determined in step (i) is used for said production.

**Figure 1**

Europäisches
Patentamt

European
Patent Office

Office européen
des brevets

## EUROPEAN SEARCH REPORT

**Application Number**

EP 24 16 5999

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Jens Uwe Pott: "Entwicklungsstrategie für zementgebundene Schäume", Dissertation, 1 January 2006 (2006-01-01), XP055261006, Universität Hannover Retrieved from the Internet: URL:https://www.baustoff.uni-hannover.de/fileadmin/baustoff/publications/Heft05-Pott-ZemSchaum.pdf [retrieved on 2016-03-24] * paragraphs [01.2], [0004], [0005] * | 1-7,9-15 | INV. G01N33/38 |
| X | BATOOL FARNAZ ET AL: "Quantification of factors influencing the thermal conductivity of cement-based foam", CEMENT AND CONCRETE COMPOSITES, vol. 91, 1 August 2018 (2018-08-01), pages 76-86, XP093196137, GB ISSN: 0958-9465, DOI: 10.1016/j.cemconcomp.2018.04.015 Retrieved from the Internet: URL:https://www.sciencedirect.com/science/article/pii/S0958946517300872> * the whole document * | 1-4,6-15 | |
| A | BATOOL FARNAZ ET AL: "Evaluation of thermal conductivity of cement-based foam reinforced with polypropylene fibers", MATERIALS AND STRUCTURES, SPRINGER NETHERLANDS, DORDRECHT, vol. 53, no. 1, 3 February 2020 (2020-02-03), XP037005912, ISSN: 1359-5997, DOI: 10.1617/S11527-020-1445-7 [retrieved on 2020-02-03] * paragraphs [02.3], [02.5], [0003] * | 1-15 | |

TECHNICAL FIELDS SEARCHED (IPC)

G01N
C04B
B01F

-/--

The present search report has been drawn up for all claims

| Place of search | Date of completion of the search | Examiner |
|---|---|---|
| The Hague | 21 August 2024 | Kraus, Leonie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EUROPÄISCHES
Patentamt

European
Patent Office

Office européen
des brevets

## EUROPEAN SEARCH REPORT

**Application Number**

EP 24 16 5999

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | CHU ZHAOXIANG ET AL: "Enhanced fractal capillary bundle model for effective thermal conductivity of composite-porous geomaterials", INTERNATIONAL COMMUNICATIONS IN HEAT AND MASS TRANSFER, PERGAMON, NEW YORK, NY, US, vol. 113, 21 February 2020 (2020-02-21), XP086115618, ISSN: 0735-1933, DOI: 10.1016/J.ICHEATMASSTRANSFER.2020.104527 [retrieved on 2020-02-21] * paragraph [05.4]; figure 15; table 1 * ----- | 1-15 | |

**TECHNICAL FIELDS SEARCHED (IPC)**

The present search report has been drawn up for all claims

| Place of search | Date of completion of the search | Examiner |
|---|---|---|
| The Hague | 21 August 2024 | Kraus, Leonie |

EPO FORM 1503 03.82 (P04C01)

**EP 4 624 924 A1**

**Patent documents cited in the description**

- WO 0170647 A
- WO 2019038105 A
- WO 2018162381 A